# EUROPÄISCHE PATENTSCHRIFT

(11) **EP 2 886 095 B1**
(45) Veröffentlichungstag und Bekanntmachung des Hinweises auf die Patenterteilung: **31.01.2018**
(21) Anmeldenummer: 13198497.3
(22) Anmeldetag: 19.12.2013
(51) Int. Cl.: A61F 13/62

(54) **Verbundstoffbahn sowie Verfahren zur Herstellung einer Verbundstoffbahn**
Composite sheet and method for making the same
Bande de matière composite ainsi que procédé destiné à la fabrication d'une bande de matière composite

(43) Veröffentlichungstag der Anmeldung: 24.06.2015
(73) Patentinhaber: Mondi AG, 1030 Wien (AT)
(72) Erfinder: Bader, Herbert, 48356 Nordwalde (DE); Großmann, Marcel, 45309 Essen (DE)
(74) Vertreter: Lorenz, Bernd Ingo Thaddeus

(56) Entgegenhaltungen:
- EP-A1- 1 736 306
- EP-A1- 2 301 502
- JP-A- 2005 022 302
- US-A1- 2008 193 709

## Beschreibung

Die Erfindung betrifft eine Verbundstoffbahn mit elastischen und unelastischen Bereichen, aus der Windelverschlusselemente abtrennbar bzw. ausstanzbar sind, umfassend eine Nonwovenbahn, welche eine erste Außenseite der Verbundstoffbahn bildet, zueinander beabstandete, elastisch dehnbare Streifen, die auf der Nonwovenbahn angeordnet sind, ein Nonwovenmaterial, welches an einer zweiten Außenseite der Verbundstoffbahn die elastisch dehnbaren Streifen abdeckt, und zumindest einen nicht elastischen Streifen aus einem Hakenmaterial, der einen Bereich zwischen zwei benachbarten elastisch dehnbaren Streifen überbrückt.

Bei der Herstellung von Verbundstoffbahnen für Windelverschlusselemente ist es aus der EP 2 301 502 A1 und aus der EP2564822 A1 bekannt, elastische Streifen zwischen zwei flächige Nonwovenbahnen einzukaschieren, wobei zwischen zwei benachbarten elastisch dehnbaren Folienstreifen wechselweise eine direkte Verbindung der beiden Nonwovenbahnen oder die Einbindung eines Verstärkungsstreifens erfolgt. Dort, wo die beiden Nonwovenbahnen direkt miteinander verbunden sind, kann das Windelverschlusselement an einem Wegwerfartikel wie einer Babywindel oder einem Hygieneprodukt für Erwachsene befestigt werden, während der Abschnitt mit dem elastisch dehnbaren Folienstreifen dem Windelverschlusselement die erforderliche Elastizität verleiht. Dort, wo der Verstärkungsstreifen angeordnet ist, kann dann nachträglich eine Hakenband aufgebracht werden, wobei der Verstärkungsstreifen dazu notwendig ist, dass das Nonwovenmaterial unter Zug nicht unkontrolliert zerreißt. Aus dem gleichen Grund ist auch eine Überlappung zwischen dem Verstärkungsstreifen und dem elastisch dehnbaren Folienstreifen vorgesehen.

Eine Verbundstoffbahn mit elastischen und unelastischen Bereichen ist auch aus der EP 1 736 306 A1 bekannt, wobei dort elastische und unelastische Bereiche durch einen entsprechenden Klebstoffauftrag gebildet werden. Der Klebstoff ist dabei dazu vorgesehen, das Material in ausreichendem Maße zu verfestigen, wobei entsprechend an elastisch dehnbaren Folienstreifen nur eine abschnittsweise Verklebung vorgesehen ist. Die nachträgliche Anordnung eines Hakenmaterials an der Außenseite kann jedoch problematisch sein, weil das Material unterhalb des Hakenbandes nur durch eine durchgehende Klebstoffschicht verstärkt ist.

Eine Verbundstoffbahn mit den eingangs beschriebenen Merkmalen ist aus der EP 2 340 796 A1 bekannt, wobei auch hier das Hakenmaterial außen auf eine von zwei flächigen Nonwovenbahnen aufgeklebt ist. Um dem Material insgesamt eine ausreichende Festigkeit zu verleihen, ist an der gegenüberliegenden Seite ein weiterer, nichtelastischer Streifen zur Verstärkung vorgesehen. Die Applikation von zwei gegenüberliegenden nichtelastischen Materialstreifen ist hinsichtlich der Materialkosten und der Verfahrensführung aufwendig.

Aus der EP 0 768 075 B1 ist eine Wegwerfwindel mit seitlichen Wiederverschlusselementen bekannt, die keine elastischen Bereiche aufweisen. Die notwendige Elastizität der gesamten Wegwerfwindel wird dadurch erreicht, dass in einem vorderen Taillenbereich ein elastisches Element angeordnet ist, während die Windelverschlusselemente in Form von seitlichen Flügeln starr sind. An den seitlichen Windelverschlusselementen ist ein Hakenmaterial vorgesehen, welches an seinen Rändern von einer Schutzschicht aus Nonwoven überdeckt wird. Die Schutzschicht aus Nonwoven wird dazu vorgesehen, um die Haken vollständig zu verdecken, um einen weichen Griffabschnitt für einen Benutzer zu bilden.

Aus der WO 99/13745 A1 ist ein Hakenmaterial bekannt, welches hakenfreie Ränder aufweist, wobei die hakenfreien Ränder von einem Deckmaterial abgedeckt sind. Die beschriebene Anordnung ist dazu vorgesehen, in Taillenbereich einer Windel angeordnet zu werden, um daran Windelverschlusselemente befestigen zu können, die ein Schlaufenmaterial aufweisen. Es ist nicht beschrieben, die aus der WO 99/13745 A1 bekannte Anordnung abschnittsweise elastisch auszuführen.

Der vorliegenden Erfindung liegt die Aufgabe zugrunde, eine Verbundstoffbahn mit elastischen und unelastischen Bereichen, aus der Windelverschlusselemente abtrennbar bzw. ausstanzbar sind, anzugeben, welche eine besonders zuverlässige Befestigung eines Hakenmaterials ermöglicht. Des Weiteren soll auch ein geeignetes Verfahren zur Herstellung einer solchen Verbundstoffbahn angegeben werden.

Gegenstand der Erfindung und Lösung der Aufgabe sind eine Verbundstoffbahn gemäß Patentanspruch 1 sowie ein Verfahren zur Herstellung der Verbundstoffbahn gemäß Patentanspruch 5.

Ausgehend von einer Verbundstoffbahn mit den eingangs beschriebenen Merkmalen ist erfindungsgemäß vorgesehen, dass das Nonwovenmaterial Ränder des Streifens aus Hakenmaterial überlappt, wobei der Streifen aus Hakenmaterial an seinen von dem Nonwovenmaterial überlappten Rändern Vorsprünge aufweist, die sich in das Nonwovenmaterial hineinerstrecken.

Durch die erfindungsgemäße Ausgestaltung werden verschiedene Vorteile erzielt. Zunächst ist das Hakenmaterial durch die Überlappung durch das Nonwovenmaterial auf verbesserte Weise in die Verbundstoffbahn integriert.

Die Ränder des Hakenmaterials sind verdeckt angeordnet, so dass diese nicht zugänglich und gut geschützt sind.

Die Elemente der Verbundstoffbahn sind üblicherweise miteinander verklebt und/oder verschweißt. Da an den überlappten Rändern des Streifens aus Hakenmaterial die Vorsprünge sich in das Nonwovenmaterial hineinerstrecken, ergibt sich zusätzlich auch eine mechanische Verbindung. Durch die in das Nonwovenmaterial eingreifenden Vorsprünge ergibt sich in der Ebene der Verbundstoffbahn eine mechanische Verbindung nach Art eines Formschlusses. Selbst bei sehr großen Belastungen kann so ein Zerreißen der Verbundstoffbahn an dem Übergang von dem elastischen Bereich zu dem unelastischen Bereich sicher verhindert werden.

Der vorliegenden Erfindung liegt die Erkenntnis zugrunde, dass bei den aus dem Stand der Technik bekannten Verbundstoffbahnen mit elastischen und unelastischen Bereichen, aus denen Windelverschlusselemente ausstanzbar sind, stets eine zusätzliche Verstärkung im Bereich des Hakenmaterials notwendig ist. Gemäß der vorliegenden Erfindung wird diese Funktion aber von dem Hakenmaterial selbst übernommen, welches einen integralen Bestandteil in dem Schichtaufbau der Verbundstoffbahn bildet.

Gemäß einer bevorzugten Ausgestaltung der Erfindung ist vorgesehen, dass die Vorsprünge sich durch das Nonwovenmaterial hindurcherstrecken. Die gesamte Dicke des Nonwovenmaterials wird dann für eine mechanische Verankerung nach Art eines Formschlusses genutzt.

Davon ausgehend können die sich durch das Nonwovenmaterial hindurcherstreckenden Vorsprünge an ihren Ende Köpfe mit einer Abwinklung und/oder Verdickung aufweisen. Zusätzlich zu einem Formschluss in der Ebene der Verbundstoffbahn wird auch senkrecht dazu eine sichere Fixierung des Nonwovenmaterials erreicht. Mit anderen Worten kann durch die Abwinklung und/oder Verdickung der Vorsprünge dann erreicht werden, dass das Nonwovenmaterial an den überlappenden Rändern nicht nach oben angehoben werden kann. Zusätzlich zu einer üblicherweise vorgesehenen Verklebung wird damit eine besonders feste und sichere Verbindung erreicht.

Das Hakenmaterial weist üblicherweise einen Trägerabschnitt und zwischen den vom Nonwovenmaterial abgedeckten Rändern frei Haken auf, die für die Verbindung mit einem geeigneten Schaufenmaterial vorgesehen sind. Auch die Vorsprünge sind vorzugsweise auf dem Trägerabschnitt angeordnet. Beispielsweise kann das gesamte Hakenmaterial aus einem thermoplastischen Kunststoff gebildet sein, wobei dann die freien Haken sowie die Vorsprünge im schmelzflüssigen Zustand aus dem Trägerabschnitt herausgezogen bzw. herausgedrückt werden.

Unabhängig von der genauen Art der Herstellung liegt das Nonwovenmaterial an den überlappten Rändern vorzugsweise auf dem Trägerabschnitt auf. Mit anderen Worten ist das an der zweiten Außenseite der Verbundstoffbahn vorgesehene Nonwovenmaterial direkt auf dem Trägerabschnitt abgelegt. Dadurch wird erreicht, dass sich die Vorsprünge weit in das Nonwovenmaterial hineinerstrecken, wobei das Nonwovenmaterial in dem überlappten Bereich auch an dem Trägerabschnitt verklebt werden kann. Zusätzlich ergibt sich der Vorteil, dass das Nonwovenmaterial an den überdeckten Rändern möglichst flach und gut geschützt ist.

Insbesondere können im Rahmen der Erfindung die freien Haken zwischen den von dem Nonwovenmaterial abgedeckten Rändern und die in den abgedeckten Bereichen angeordneten Vorsprüngen eine übereinstimmende Form aufweisen.

Die Vorsprünge und die Haken unterscheiden sich dann im Wesentlichen nur dadurch, dass die Vorsprünge von dem Nonwovenmaterial umgeben sind, während die Haken frei sind. Grundsätzlich können auch die Vorsprünge in einem gewissen Maße noch für eine Verhakung mit einem Schlaufenmaterial vorgesehen sein.

Im Rahmen der beschriebenen Ausgestaltung können die Köpfe der freien Haken sowie der Vorsprünge in Form einer Abwinklung und/oder Verdickung vor oder nach dem Verbinden der einzelnen Schichten der Verbundstoffbahn erzeugt werden. Wenn bereits vor der Verbindung mit dem Nonwovenmaterial unter Erzeugung eines Formschlusses die Vorsprünge mit Köpfen versehen sind, muss die Nonwovenbahn über diese Köpfe geführt werden, wozu elastisch nachgiebige Andruckrollen oder dergleichen eingesetzt werden können.

Gemäß einer bevorzugten Ausgestaltung der Erfindung ist jedoch vorgesehen, dass Köpfe in Form einer Abwinklung und/oder Verdickung erst nach dem Zusammenfügen der einzelnen Bestandteile der Verbundstoffbahn gebildet werden. Es wird dann ein nicht elastischer Streifen bereitgestellt, der zunächst nur über Stiele verfügt, die sich von dem Trägerabschnitt wegerstrecken. Wenn dann das Nonwovenmaterial aufgelegt wird, kann dieses an den Rändern des nicht elastischen Streifens leicht von den Stielen durchstochen werden. Erst danach werden dann im Rahmen der beschriebenen Variante an den Enden der Stiele Köpfe mit einer Abwinklung und/oder Verdickung gebildet, wodurch sich aus den Stielen einerseits die freien Haken zwischen den überlappten Rändern sowie die formschlüssig mit dem Nonwovenmaterial verbundenen Vorsprünge ergeben.

Die Erzeugung der Köpfe kann insbesondere durch die Einwirkung von Druck und Temperatur erfolgen. Dabei kann es auch zweckmäßig sein, wenn durch eine geeignete Ausgestaltung des für die Umformung vorgesehenen Werkzeuges die Stiele für die Bildung der formschlüssig verbundenen Vorsprünge stärker verformt werden. Es kann so eine besonders innige und zuverlässige Verbindung zwischen dem nicht elastischen Streifen aus Hakenmaterial und dem an den Rändern überlappend angeordneten Nonwovenmaterial erreicht werden.

Der Überlappungsbereich zwischen dem Streifen aus Hakenmaterial und dem Nonwovenmaterial ist so zu wählen, dass eine sichere Befestigung möglich ist. Der Überlappungsbereich kann beispielsweise zwischen 2 mm und 30 mm, vorzugsweise zwischen 8 mm und 20 mm liegen.

Die elastisch dehnbaren Streifen werden vorzugsweise von einer elastisch dehnbaren Folie gebildet. Das die elastisch dehnbaren Streifen bzw. Folienstreifen an der zweiten Seite der Verbundstoffbahn abdeckende Nonwovenmaterial liegt zweckmäßigerweise in Form von voneinander beabstandeten Nonwovenstreifen vor. Im Rahmen der Erfindung können die elastisch dehnbaren Streifen entweder direkt an den nicht elastischen Streifen aus Hakenmaterial herangeführt sein oder auch in einem Abstand von typischerweise einigen Millimetern zu dem nicht elastischen Streifen aus Hakenmaterial enden. Erfindungsgemäß ist der Übergangsbereich an der zweiten Außenseite durch das dort vorhandene Nonwovenmaterial abgedeckt und geschützt. Das Nonwovenmaterial erstreckt sich also in Richtung des nicht elastischen Streifens aus Hakenmaterial über die elastisch dehnbaren Streifen hinaus.

Gemäß einer bevorzugten Ausgestaltung der Erfindung ist das Hakenmaterial unmittelbar auf der flächigen Nonwovenbahn befestigt. Auch die elastisch dehnbaren Folienstreifen können direkt auf der Nonwovenbahn befestigt sein.

Für die Befestigung kommen grundsätzlich unterschiedliche Möglichkeiten in Betracht. Die Befestigung kann beispielsweise durch Klebstoff erfolgen, der wahlweise vollflächig oder nur in Bereichen aufgetragen werden kann. Je nach Art der Verklebung und abhängig von den Materialien sowie Belastungen kann eine streifenförmige Verklebung ausreichend sein, um das Hakenmaterial sicher zu befestigen. Dabei ist auch zu berücksichtigen, dass durch die formschlüssige Verbindung des Hakenmaterials mit dem Nonwovenmaterial an der zweiten Außenseite eine erhöhte Festigkeit erreicht wird.

Durch eine streifenförmige Verklebung kann grundsätzlich Klebstoff eingespart werden, wodurch sich eine Kostenreduktion ergibt. Dabei ist auch zu berücksichtigen, dass geeignete Klebstoffe nicht nur relativ teuer sind, sondern auch die Elastizität beeinträchtigen können. Dieser negative Einfluss des Klebstoffes wird durch einen streifenförmigen Auftrag unterhalb der elastisch dehnbaren Streifen reduziert. Vorteilhaft ist insbesondere ein streifenförmiger Klebstoffauftrag parallel zu der Erstreckung des elastisch dehnbaren Streifens, wobei die einzelnen aus der Verbundstoffbahn gebildeten Windelverschlusselemente bei ihrer Benutzung quer dazu gedehnt werden.

Die elastisch dehnbaren Streifen bestehen vorzugsweise aus einer Folie aus einem geeigneten thermoplastischen Elastomer, wobei insbesondere ein Polymer aus der Gruppe der Styrol-Butadien-Styrol-Block-Copolymere (SBS), Styrol-Isopren-Styrol-Block-Copolymere (SIS), Styrol-Ethen-Buten-Styrol-BlockCopolymer (SEBS), elastischen Polyethylen-Copolymere, elastische Polypropylen-Copolymere, elastischen Polyurethan-Copolymere, elastische Polyamid-Copolymere, oder einer Mischung dieser Polymere geeignet ist. Neben einer Verwendung von Monofolien können auch coextrudierte Folien eingesetzt werde, wobei auch coextrudierte Folien mit mehreren identischen Schichten geeignet sind. Besonders geeignet sind Folienstreifen mit einer Dicke zwischen 10 und 130 µm.

Das die elastischen Streifen an der zweiten Außenseite abdeckende Nonwovenmaterial sowie die an der gegenüberliegenden Seite der Verbundstoffbahn angeordnete Nonwovenbahn sind üblicherweise selbst nicht elastisch, aber im ausreichenden Maße dehnbar, um die elastischen Bereiche der Verbundstoffbahn bilden zu können. Die notwendige Dehnbarkeit kann aber auch durch eine Aktivierung der Verbundstoffbahn durch ein erstmaliges Dehnen erfolgen, bei dem die Nonwovenschichten auch zu einem Teil zerstört, d. h. zerrissen werden. Das Nonwoven verleiht der Verbundstoffbahn eine weiche textile Haptik.

Gegenstand der Erfindung ist auch ein Verfahren zur Herstellung der beschriebenen Verbundstoffbahn, wobei eine Nonwovenbahn, elastisch dehnbare Streifen, Streifen eines Nonwovenmaterials und ein nicht elastischer Streifen mit Vorsprüngen an seinen Rändern bereitgestellt werden. Der nicht elastische Streifen wird mittelbar oder unmittelbar auf die Nonwovenbahn aufgelegt, wobei die Streifen des Nonwovenmaterials derart überlappend auf den Rändern des nicht elastischen Streifens angeordnet werden, dass die Vorsprünge sich in das Nowovenmaterial hineinerstrecken. Wie zuvor beschrieben, können die Vorsprünge Stiele und an ihren Enden Köpfe mit einer Abwinklung und/oder Verdickung aufweisen.

Die Streifen des Nonwovenmaterials können entweder vor oder nach dem nicht elastischen Streifen zugeführt werden.

So können gemäß einer ersten Variante der Erfindung zunächst die Nonwovenbahn, die elastisch dehnbaren Streifen und die Streifen des Nonwovenmaterials miteinander verbunden werden, wobei zwischen den elastischen Streifen ein Abstand verbleibt und wobei sich die Streifen des Nonwovenmaterials dort mit freien Enden über die elastisch dehnbaren Streifen hinauserstrecken. Diese freien Enden werden dann auseinandergeklappt, um den nicht elastischen Streifen aus Hakenmaterial bzw. den nicht elastischen Streifen, aus dem das Hakenmaterial gebildet wird, zwischen den elastisch dehnbaren Streifen anordnen zu können. Die aufgeklappten Abschnitte des Nonwovenmaterials werden dann zurück auf das Hakenmaterial gelegt, wodurch sich in dem überlappenden Bereich eine formschlüssige Verbindung mit den Vorsprüngen ergibt. Sofern sich die Vorsprünge durch das Nonwovenmaterial hindurcherstrecken, können die Vorsprünge dann in der beschriebenen Weise durch Einwirkung von Druck und/oder Temperatur umgeformt werden, um auch in vertikaler Richtung einen Formschluss zu erhalten. Gleichzeitig kann auch zwischen den Rändern des Streifens aus Hakenmaterial in der beschriebenen Weise freie Haken gebildet werden.

Wenn zunächst das Hakenmaterial und dann die Streifen des Nonwovenmaterials zugeführt werden, können die Streifen des Nonwovenmaterials direkt überlappend auf das Hakenmaterial sowie die elastisch dehnbaren Streifen aufgelegt werden, wobei dann ein nachträgliches Falten nicht mehr notwendig ist.

Ein weiterer Aspekt der vorliegenden Erfindung, dem eine eigenständige erfinderische Bedeutung zukommt, betrifft die Bildung eines Hakenmaterials. Dieses Hakenmaterial ist in besonderem Maße für die zuvor beschriebene Verbundstoffbahn geeignet, grundsätzlich aber auch in anderen Bereichen einsetzbar.

Zur Bildung des Hakenmaterials wird eine Polymerschmelze von einer Breitschlitzdüse in einen Spalt zwischen einer Walze und einem abschnittsweise entlang der Oberfläche der Walze geführten Band gegeben, wobei die Walze und/oder das Band eine für die Bildung von Haken vorgesehene Strukturierung aufweisen. Je nach Ausgestaltung des Verfahrens können entweder vollständige Haken mit Stielen und endseitigen Köpfen oder zunächst nur die Stiele der Haken bzw. der Vorsprünge gebildet werden, wobei dann die Köpfe nachträglich erzeugt werden.

Unter Haken werden im Rahmen der Erfindung Elemente verstanden, die dazu geeignet sind, sich in einem zugeordneten Material, beispielsweise einem mit freien Schlaufen gewirkten Textil oder einem Schlaufen aufweisenden Non-woven, zu verhaken. Die Haken können dabei sowohl an den Stielen umgebogene Enden oder auch pilzförmige Verbreiterungen als Köpfe aufweisen.

Da eine zunächst noch flüssige Polymerschmelze aus der Schlitzdüse auf den Spalt gegeben wird, kann die Ausformung der Haken des Hakenmaterials sehr frei variiert werden. So kann beispielsweise die zur Bildung der Haken vorgesehene Strukturierung an Abschnitten des Bandes bzw. der Walze unterbrochen sein, um dort keine Haken zu bilden. Insbesondere kann die Strukturierung in Querrichtung und/oder Umfangsrichtung unterbrochen sein, wobei dann durch eine ebene Oberfläche hakenfreie Bereiche erzeugt werden.

Neben der Erzeugung von Bereichen mit Haken bzw. Vorsprüngen sowie hakenfreien Bereichen kann bei dem beschriebenen Verfahren auch durch eine entsprechende Konturierung des Bandes und/oder der Walze der Walzenspalt und damit die lokale Dicke des erzeugten Hakenmaterials eingestellt werden.

Wenn gemäß einer ersten Variante zur Bildung des Hakenmaterials ein glattes auch als Sleeve bezeichnetes Band und eine strukturierte Walze (Chill-Roll) eingesetzt werden, werden die Stiele der Haken bzw. Vorsprünge durch die strukturierte, üblicherweise gekühlte Walze (Chill-Roll) erzeugt.

Im Rahmen der Strukturierung werden Vertiefungen bzw. Kavitäten zur Ausformung der Stiele der Haken bzw. Vorsprünge in der Walze gebildet. Die Walzenoberfläche kann dazu beispielsweise mit einem Ätzverfahren oder durch Einsatz eines Lasers bearbeitet werden. Die Anzahl und Verteilung der Kavitäten kann variabel an den jeweiligen Anwendungsfall des zu bildenden Hakenbandes angepasst werden.

Das Band kann beispielsweise vollständig nahtlos durch einen galvanischen Prozess erzeugt werden. Ein solches Band, welches abhängig von der Anwendung auch als Glättband bezeichnet wird, wird üblicherweise zwischen zwei temperierten Walzen gespannt, von denen mindestens eine angetrieben werden kann. Das gespannte Band wird üblicherweise durch einen verfahrbaren Walzenstuhl gegen die strukturierte, gekühlte Walze gepresst. Durch das Anpressen des Bandes in einem üblicherweise variabel einstellbaren Umschlingungswinkel wird erreicht, dass die zunächst noch flüssige Polymerschmelze die Vertiefungen bzw. Kavitäten der strukturierten Walze ausfüllt. Auf eine zusätzliche Entlüftung kann dabei in der Regel verzichtet werden, wobei auch eine mehrschichtige Ausgestaltung der Walze zur Entlüftung nicht notwendig ist. Durch den Kontakt mit dem umlaufenden Band während der Ausbildung der Stiele der Haken bzw. Vorsprünge kann die Abkühlung besser kontrolliert werden, wodurch eine Fertigung auch bei einer sehr hohen Produktionsgeschwindigkeit möglich ist. Durch den Einsatz des beschriebenen Bandes kann auch eine besonders gleichmäßige, glatte und hochwertige Rückseite des Hakenmaterials erzeugt werden.

Wie bereits zuvor erläutert, kann die Strukturierung in Längsrichtung, Querrichtung oder auch in einer beliebigen Richtung unterbrochen sein, um hakenfreie Bereiche zu erzeugen.

Gemäß einer zweiten Variante zur Bildung des Hakenmaterials werden eine glatte Walze und ein strukturiertes bzw. perforiertes Band eingesetzt. Bei einem perforierten Band ist unter allen Umständen eine ausreichende Entlüftung der einzelnen Löcher zur Erzeugung der Stiele der Haken bzw. Vorsprünge gewährleistet. Durch die Dicke des Bandes kann auch die Länge der auszuformenden Stiele eingestellt werden. Alternativ kann die Länge der Stiele auch durch den Anpressdruck des Bandes an die Walze eingestellt werden, wobei dann bei einem reduzierten Anpressdruck die Löcher der Perforation nur teilweise ausgefüllt werden. Durch eine Variation des Anpressdruckes kann dabei ohne einen Austausch des Bandes die Höhe der Stiele der Haken verändert werden.

Das Hakenmaterial kann je nach Anforderung und Einsatzbereich aus unterschiedlichen Materialien sowohl einschichtig als auch mehrschichtig aufgebaut sein. Bei einem einschichtigen Aufbau sind insbesondere polyolefinische Materialien wie Polyethylen (PE), Polypropylen (PP), Mischungen der genannten Polymere sowie ein Copolymer von Polyethylen und Polypropylen geeignet. Besonders bevorzugt werden aber steife PP-Typen eingesetzt, die sowohl als Homo- oder als Copolymere vorliegen können. Darüber hinaus sind aber auch andere Polymere für einen einschichtigen oder mehrschichtigen Aufbau geeignet. Vorteilhaft sind insbesondere auch vergleichsweise steife Materialien wie Cycloolefin-Copolymer (COC) Polyester wie beispielsweise Polyethylenterephthalat (PET), Polyamide (PA) wie beispielsweise PA 6 und PA 6,6 oder auch Polymethylmethacrylat (PMMA).

Durch einen mehrschichtigen Aufbau des Hakenbandes können die zum Teil einander entgegenstehenden Anforderungen von z. B. Steifigkeit, Fließfähigkeit während der Herstellung und Kompatibilität zu den im Laminat benachbarten Schichten auf vorteilhafte Weise erfüllt werden. Gemäß einer ersten Variante sind weitgehend typenreine Aufbauten oder zumindest Aufbauten vollständig aus Polyolefin möglich, wodurch insbesondere auch eine Wiederverwertung erleichtert wird. Beispielsweise kann bei einem dreischichtigen Aufbau für alle Schichten Polypropylen (PP) vorgesehen sein, wobei jedoch die Schicht, aus der die Haken bzw. Vorsprünge gebildet werden sollen, speziell hinsichtlich eines verbesserten Prägeverhaltens mit einem hohen Fließindex (MFI) ausgewählt werden, während die übrigen Schichten gezielt hinsichtlich der erforderlichen Steifigkeit des Hakenbandes optimiert sind.

Neben einem solchen typenreinen Aufbau ist auch ein mehrschichtiger Aufbau aus verschiedenen Materialtypen möglich, wobei in den Außenschichten bevorzugt Materialien auf polyolefinischer Basis, insbesondere PP, eingesetzt werden. Bei einem Aufbau mit zumindest drei Schichten kann eine innenliegende Kernschicht hinsichtlich einer maximalen Steifigkeit ausgewählt sein. Während bei einem fünfschichtigen Aufbau zwischen der Kernschicht und den Außenschichten jeweils eine Haftvermittlerschicht angeordnet wird, kann bei einem dreischichtigen Aufbau eine ausreichende Verbindung der Schichten untereinander durch funktionalisierte bzw. modifizierte Polyolefine erreicht werden.

Die Dicke des Hakenmaterials beträgt unterhalb der Haken bzw. Vorsprünge typischerweise zwischen 20 und 180 µm, besonders bevorzugt zwischen 70 und 140 µm.

Die vorliegende Erfindung wird im Folgenden anhand von Zeichnungen erläutert, welche lediglich Ausführungsbeispiele darstellen. Es zeigen in einer schematischen Darstellung
- Fig. 1: die Bestandteile, aus der eine Verbundstoffbahn gebildet wird,
- Fig. 2: die miteinander verbundenen Bestandteile,

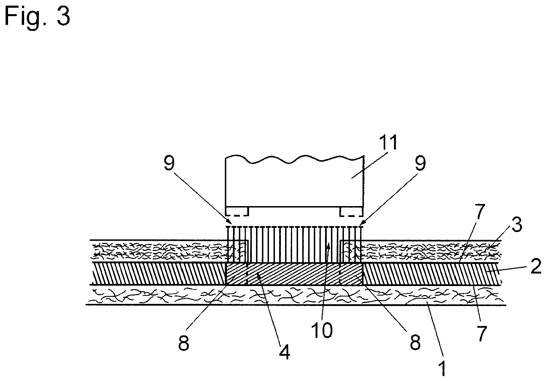

- Fig. 3: ein Verfahrensschritt zur Erzeugung von Haken,
- Fig. 4 bis Fig. 6: eine Variante des Verfahrens zur Herstellung der Verbundstoffbahn,
- Fig. 7: eine alternative Ausgestaltung der Verbundstoffbahn und
- Fig. 8: eine Draufsicht auf eine Verbundstoffbahn mit einem Bestandsmuster zum Abtrennen einzelner Verschlusselemente,
- Fig. 9: eine weitere alternative Ausgestaltung der Verbundstoffbahn.

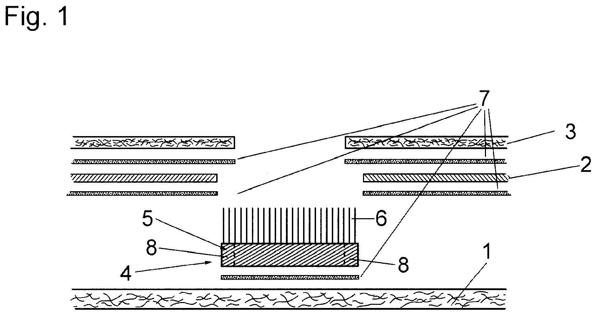

Die Fig. 1 zeigt in einem Querschnitt die einzelnen Bestandteile, aus der eine Verbundstoffbahn mit elastischen und unelastischen Bereichen gebildet wird, aus der Windelverschlusselemente ausstanzbar bzw. abtrennbar sind. Die Verbundstoffbahn umfasst eine flächige Nonwovenbahn 1, welche eine erste Außenseite der Verbundstoffbahn bildet. Um die elastischen Bereiche der Verbundstoffbahn zu erzeugen, sind parallel zueinander beabstandete, elastisch dehnbare Folienstreifen 2 auf der Nonwovenbahn 1 angeordnet, wobei diese Folienstreifen 2 von einem Nonwovenmaterial 3 abgedeckt sind. Das in Form von Nonwovenstreifen angeordnete Nonwovenmaterial 3 deckt die Folienstreifen 2 vollständig ab und bildet bereichsweise eine zweite Außenseite der Verbundstoffbahn. Zusätzlich ist ein nicht elastischer Streifen 4 vorgesehen, aus dem ein Hakenmaterial gebildet wird. Der nicht elastische Streifen 4 weist einen Trägerabschnitt 5 und nach oben abstehende Stiele 6 auf.

Die beschriebenen Bestandteile können auf unterschiedliche Weise miteinander verbunden werden. Insbesondere kann eine vollflächige oder abschnittsweise Verklebung mittels Klebstoff 7 oder auch ein Ultraschallschweißen erfolgen. Bei dem lediglich abschnittsweise vorgesehenen Verkleben erfolgt die Verbindung zweckmäßigerweise durch Klebstoffstreifen, die sich entlang der Längsrichtung der beschriebenen streifenförmigen Elemente erstrecken.

In der Fig. 1 sind die einzelnen Bestandteile der Verbundstoffbahn dargestellt, wobei lediglich exemplarisch ein flächiger Auftrag von Klebstoff 7 gezeigt ist.

In der Fig. 1 ist zu erkennen, dass die beiden elastisch dehnbaren Folienstreifen 2 seitlich des nicht elastischen Streifens 4 angeordnet werden, während das Nonwovenmaterial 3 Ränder 8 des nicht elastischen Streifens 4 überlappt.

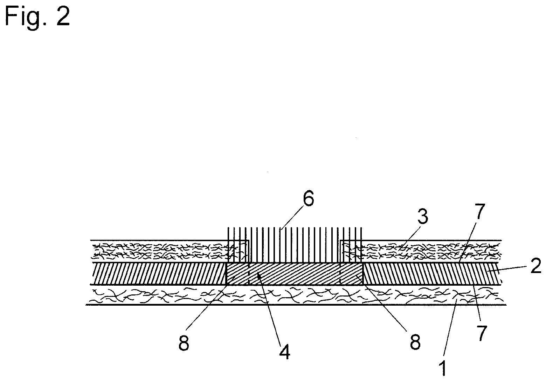

Wenn zunächst der nicht elastische Streifen 4 auf der Nonwovenbahn 1 angeordnet wird, bevor die elastisch dehnbaren Folienstreifen 2 und die Streifen von Nonwovenmaterial 3 zugeführt werden, wird das Nonwovenmaterial 3 an den überlappenden Rändern 8 unmittelbar bei seiner Zuführung von den Stielen 6 an den Rändern 8 durchstochen, wodurch das Nonwovenmaterial 3 durch die Stiele 6 gehalten wird (Fig. 2).

Die Stiele 6 stellen dann an den Rändern 8 Vorsprünge 9 dar, welche sich in das Nonwovenmaterial 3 hinein und durch das Nowovenmaterial 3 hindurcherstrecken.

Um zwischen den überlappten Rändern 8 freie Haken 10 zu bilden, werden die Stiele 6 mit einem geeigneten Werkzeug 11 unter Einwirkung von Druck und Temperatur umgeformt, wobei Köpfe mit einer Abwinklung und/oder Verdickung erzeugt werden. Auch die Stiele 6 an den überlappten Rändern 8 werden in gleicher Weise umgeformt, wobei das Nonwovenmaterial 3 nicht nur in der Ebene der Verbundstoffbahn sondern auch senkrecht dazu in einem Formschluss fixiert wird. Im Rahmen der Erfindung kann auch ein Werkzeug 11 eingesetzt werden, welches die Vorsprünge 9 bildenden Stiele 6 stärker verformt als die Stiele 6, aus denen die freien Haken 10 gebildet werden.

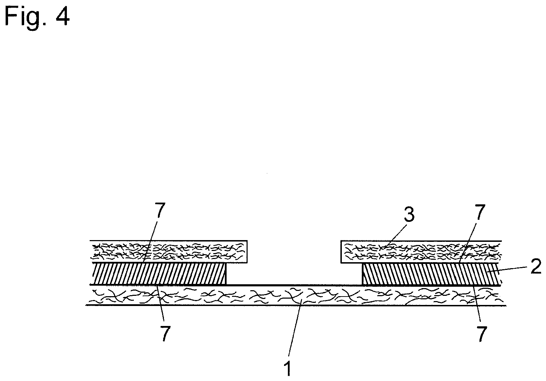

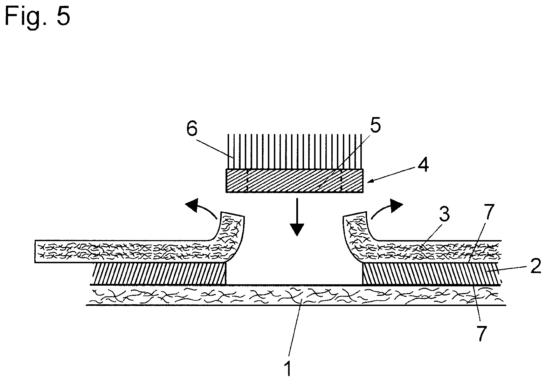

Die Figuren 4 bis 6 zeigen eine alternative Vorgehensweise bei der Herstellung der Verbundstoffbahn, wobei zunächst die Nowovenbahn 1, die elastischen Foleinstreifen 2 und die Streifen des Nonwovenmaterials 3 miteinander, beispielsweise durch Klebstoff 7, verbunden werden. Wie auch in der Fig. 1 dargestellt, erstrecken sich die Streifen des Nowovenmaterials 3 über die Folienstreifen 2 hinaus. Die Anordnung des nicht elastischen Streifens 4 erfolgt dann durch ein Zurückfalten der freien seitlichen Enden des Nonwovenmaterials 3 (Fig. 5).

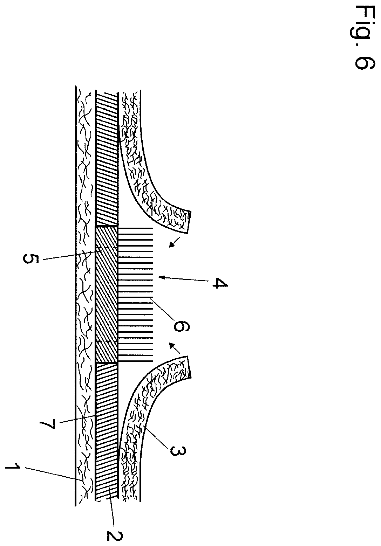

Nach dem Zurückfalten des Nonwovenmaterials (Fig. 6) ergibt sich auch bei dieser alternativen Verfahrensführung die Anordnung gemäß der Fig. 2, wobei auch dann nachfolgend eine Umformung der Stiele 6 erfolgt.

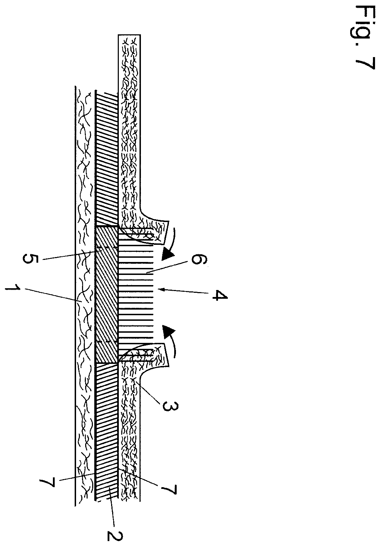

Die Fig. 7 zeigt eine weitere alternative Ausgestaltung der Erfindung, wobei die Stiele 6 an den überlappten Rändern 8 eine geringere Länge aufweisen. Die von den Stielen 6 an den überlappenden Rändern 8 gebildeten Vorsprünge 9 erstrecken sich dann nur in das Nonwovenmaterial 3 hinein aber nicht durch das gesamte Nonwovenmaterial 3 hindurch. Dennoch wird zumindest in der Ebene der Verbundstoffbahn eine zuverlässige Fixierung erreicht, wobei das Nonwovenmaterial 3 auch an dem Trägerabschnitt 5 des nicht elastischen Streifens 4 verklebt sein kann.

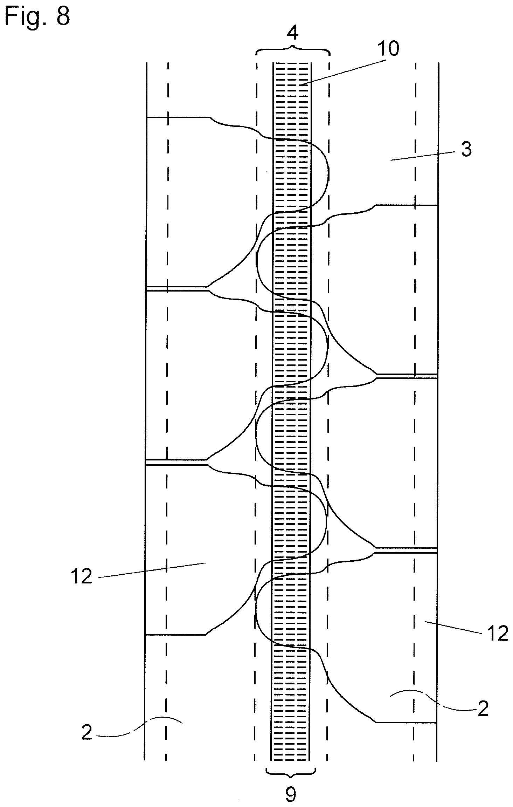

Die Fig. 8 zeigt ein mögliches Schnitt- bzw. Stanzmuster zur Erzeugung einzelner Verschlusselemente 12. In der Ausgestaltung gemäß der Fig. 8 weist jedes Verschlusselement 12 den mit Haken 10 versehenen Bereich des nicht elastischen Streifens 4 über seine gesamte Breite auf. Zusätzlich wird ein freies Ende des Verschlusselementes 12 neben den Haken 10 von einem Abschnitt der Verbundstoffbahn gebildet, welcher die Vorsprünge 9 umfasst. Insbesondere wenn sich die Vorsprünge 9 durch das Nonwovenmaterial 3 hindurcherstrecken, kann dort bei der Herstellung eine stärkere Umformung mit dem Werkzeug 11 vorgesehen sein, um einen möglichst gleichmäßigen, glatten Griffabschnitt für einen Benutzer zu erzeugen.

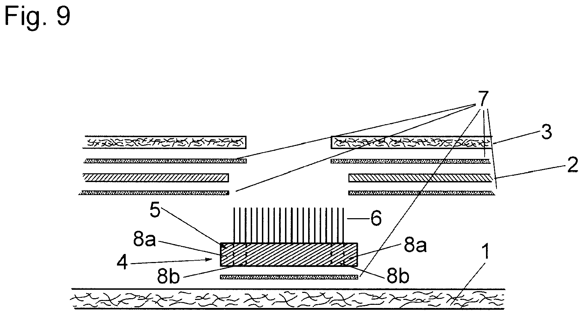

Die Fig. 9 zeigt ausgehend von der Fig. 1 eine weitere alternative Ausgestaltung der Verbundstoffbahn, bei der ein erster von dem Nonwovenmaterial 3 abgedeckter äußerer Randabschnitt 8a frei von Haken 10 bzw. Stielen 6 ist. Während die elastisch dehnbaren Folienstreifen 2 an dem äußeren Randabschnitt 8a enden, erstreckt sich das Nonwovenmaterial 3 darüber hinaus bis zu einem zweiten, weiter innen liegenden Randabschnitt 8b, der nach oben abstehende Stiele 6 aufweist.

## Patentansprüche

1. Verbundstoffbahn mit elastischen und unelastischen Bereichen, aus der Windelverschlusselemente abtrennbar bzw. ausstanzbar sind, umfassend eine Nonwovenbahn (1), welche eine erste Außenseite der Verbundstoffbahn bildet, zueinander beabstandete, elastisch dehnbare Streifen, die auf der Nonwovenbahn (1) angeordnet sind, ein Nonwovenmaterial (3), welches an einer zweiten Außenseite der Verbundstoffbahn die elastisch dehnbaren Streifen abdeckt, und zumindest einem nicht elastischen Streifen (4) aus Hakenmaterial, der einen Bereich zwischen zwei benachbarten elastisch dehnbaren Streifen überbrückt, **dadurch gekennzeichnet, dass** das Nonwovenmaterial (3) Ränder (8) des Streifens (4) aus Hakenmaterial überlappt, und dass der Streifen (4) aus Hakenmaterial an seinen von dem Nonwovenmaterial (3) überlappten Rändern (8) Vorsprünge (9) aufweist, die sich in das Nonwovenmaterial (3) hineinerstrecken.

2. Verbundstoffbahn nach Anspruch 1, **dadurch gekennzeichnet, dass** die Vorsprünge (9) sich durch das Nonwovenmaterial (3) hindurcherstrecken.

3. Verbundstoffbahn nach Anspruch 2, **dadurch gekennzeichnet, dass** die Vorsprünge (9) an ihren Enden Köpfe mit einer Abwinklung und/oder Verdickung aufweisen.

4. Verbundstoffbahn nach einem der Ansprüche 1 bis 3, **dadurch gekennzeichnet, dass** die Vorsprünge (9) an einem Trägerabschnitt (5) des Hakenmaterials angeordnet sind, wobei das Nonwovenmaterial (3) an den überlappten Rändern (8) auf dem Trägerabschnitt (5) aufliegt.

5. Verbundstoffbahn nach einem der Ansprüche 1 bis 4, **dadurch gekennzeichnet, dass** der Streifen (4) aus Hakenmaterial zwischen den von dem Nonwovenmaterial (3) abgedeckten Rändern (8) freie Haken (10) aufweist, wobei die freien Haken (10) und die Vorsprünge (9) eine übereinstimmende Form aufweisen.

6. Verfahren zur Herstellung einer Verbundstoffbahn nach einem der Ansprüche 1 bis 5,
wobei eine Nonwovenbahn (1) elastisch dehnbare Streifen, Streifen eines Nonwovenmaterials (3) und einen nicht elastischen Streifen (4) mit Vorsprüngen (9) an seinen Rändern (8) bereitgestellt werden,
wobei der nicht elastische Streifen (4) auf die Nonwovenbahn (1) aufgelegt wird,
wobei die Streifen des Nonwovenmaterials (3) derart überlappend auf den Rändern (8) des nicht elastischen Streifens (4) angeordnet werden, dass die Vorsprünge (9) sich in das Nonwovenmaterial (3) hineinerstrecken.

7. Verfahren nach Anspruch 6, wobei der nicht elastische Streifen (4) mit Vorsprüngen (9) in Form von Stielen (6) bereitgestellt wird, wobei die Streifen des Nonwovenmaterials (3) derart überlappend auf den Rändern (8) des nicht elastischen Streifens (4) angeordnet werden, dass die Stiele (6) sich durch das Nonwovenmaterial (3) hindurcherstrecken.

8. Verfahren nach Anspruch 7, wobei die Stiele (6) an den Rändern (8) des nicht elastischen Streifens (4) nach der überlappenden Anordnung des Nonwovenmaterials (3) an ihren Enden abgewinkelt und/oder mit einer Verdickung versehen werden.

9. Verfahren nach Anspruch 8, wobei die Stiele (6) durch Einwirkung von Druck und/oder Temperatur umgeformt werden.

10. Verfahren nach Anspruch 8 oder 9, wobei zwischen den Rändern (8) des nicht elastischen Streifens (4) weitere Stiele (6) angeordnet sind, die zu freien Haken (10) umgeformt werden.

## Claims

1. A composite material web with elastic and non-elastic areas, from which nappy closure elements can be cut off or punched out off, comprising a nonwoven web (1) forming a first outer side of the composite material web, elastically extendable strips spaced-apart from each other and arranged on the nonwoven web (1), a nonwoven material (3) covering the elastically extendable strips on the second outer side of the composite material web and at least one non-elastic strip (4) from a hook material, which bridges an area between two adjacent elastically extendable strips, **characterised in that** the nonwoven material (3) overlaps edges (8) of the strips (4) from the hook material, and **in that** the strip (4) from the hook material comprises projections (9) on its edges (8) which are overlapped by the nonwoven material (3), wherein the projections extend into the non-woven material (3).

2. The composite material web according to claim 1, **characterised in that** the projections (9) extend right through the nonwoven material (3).

3. The composite material web according to claim 2, **characterised in that** at their ends the projections (9) comprise heads which are angled off and/or thickened.

4. The composite material web according to one of claims 1 to 3, **characterised in that** the projections (9) are arranged on a carrier section (5) of the hook material, wherein the nonwoven material (3) rests on the carrier section (5) at the overlapped edges (8).

5. The composite material web according to one of claims 1 to 4, **characterised in that** the strip 4 from the hook material comprises free hooks (10) between the edges (8) covered by the nonwoven material (3), wherein the free hooks (10) and the projections (9) have a concordant shape.

6. A method for producing a composite material web according to one of claims 1 to 5,
wherein a nonwoven web (1), elastically extendable strips, strips of a nonwoven material (3) and a non-elastic strip (4) with projections (9) at its edges (8) are provided,
wherein the non-elastic strip (4) is placed onto the nonwoven web (1),
wherein the strips of the nonwoven material (3) are arranged on the edges (8) of the non-elastic strip (7) in an overlapping manner such that the projections (9) extend into the nonwoven material (3).

7. The method according to claim 6, wherein the non-elastic strip (4) is provided with projections (9) in the form of stems (6), wherein the strips of the non-woven material (3) are arranged at the edges (8) of the non-elastic strip (4) in an overlapping manner such that the stems (6) extend right the nonwoven material (3).

8. The method according to claim 7, wherein the stems (6) at the edges (8) of the non-elastic strip (4), following the overlapping arrangement of the nonwoven material (3), are angled off and/or provided with a thickening at their ends.

9. The method according to claim 8, wherein the stems (6) are reshaped under the influence of pressure and/or temperature.

10. The method according to claim 8 or 9, wherein further stems (6) are arranged between the edges (8) of the non-elastic strip (4), which are reshaped to form free hooks (10).

## Revendications

1. Bande de matière composite avec des zones élastiques et non élastiques, à partir de laquelle des éléments de fermeture de couches sont sectionnables ou découpables, comprenant une bande de non-tissé (1), laquelle forme une première face extérieure de la bande de matière composite, des rubans élastiquement étirables écartés les uns des autres, qui sont placés sur la bande de non-tissé (1), une matière non-tissée (3), qui sur une deuxième face extérieure de la bande de matière composite recouvre les rubans élastiquement étirables et au moins un ruban (4) non élastique, en matière à crochets qui chevauche une zone entre deux rubans élastiquement étirables voisins, **caractérisée en ce que** la matière non-tissée (3) chevauche des bords (8) du ruban (4) en matière à crochets et **en ce que** sur ses bords (8) chevauchés par la matière non-tissée (3), le ruban (4) en matière à crochets comporte des saillies (9) qui s'étendent à l'intérieur de la matière non-tissée (3).

2. Bande de matière composite selon la revendication 1, **caractérisée en ce que** les saillies (9) s'étendent à travers la matière non-tissée (3).

3. Bande de matière composite selon la revendication 2, **caractérisée en ce que** sur leurs extrémités, les saillies (9) comportent de têtes avec un coude et/ou un épaississement.

4. Bande de matière composite selon l'une quelconque des revendications 1 à 3, **caractérisée en ce que** les saillies (9) sont placées sur une partie support (5) de la matière à crochets, la matière non-tissée (3) reposant par les bords (8) chevauchés sur la partie support (5).

5. Bande de matière composite selon l'une quelconque des revendications 1 à 4, **caractérisée en ce qu'**entre les bords (8) recouverts par la matière non-tissée (3), le ruban (4) en matière à crochets comporte des crochets (10) libres, les crochets (10) libres et les saillies (9) présentant une forme concordante.

6. Procédé destiné à fabriquer une bande de matière composite selon l'une quelconque des revendications 1 à 5,
lors duquel on met à disposition une bande de non-tissé (1), de rubans élastiquement étirables, des rubans d'une matière non-tissée (3) et un ruban (4) non élastique avec des saillies (9) sur ses bords (8),
lors duquel on pose le ruban (4) non élastique sur la bande de non-tissé (1),
lors duquel on place les rubans de la matière non-tissée (3) en chevauchement sur les bords (8) du ruban (4) non élastique, de telle sorte que les saillies (9) s'étendent à l'intérieur de la matière non-tissée (3).

7. Procédé selon la revendication 6, lors duquel on met à disposition le ruban (4) non élastique avec des saillies (9) sous la forme de tiges (6), lors duquel on place les rubans de la matière non-tissée (3) en chevauchement sur les bords (8) du ruban (4) non élastique, de telle sorte que les tiges (6) s'étendant à travers la matière non-tissée (3).

8. Procédé selon la revendication 7, lors duquel, après le placement en chevauchement de la matière non-tissée (3), on coude sur leur extrémité et/on munit d'un épaississement les tiges (6) sur les bords (8) du ruban (4) non élastique.

9. Procédé selon la revendication 8, lors duquel on refaçonne les tiges (6) sous l'effet de pression et/ou de température.

10. Procédé selon la revendication 8 ou 9, lors duquel, on place entre les bords (8) du ruban (4) non élastique des tiges (6) supplémentaires, que l'on refaçonne en crochets (10) libres.
